**Europäisches Patentamt**

**European Patent Office**

**Office européen des brevets**

(19)

(11) Publication number: **0 069 407**
**B1**

(12) # EUROPEAN PATENT SPECIFICATION

(45) Date of publication of patent specification: **07.12.88**

(51) Int. Cl.⁴: **A 61 K 39/245**

(21) Application number: **82200705.0**

(22) Date of filing: **09.06.82**

(54) Method of immunizing pigs against Aujeszky's disease.

(30) Priority: **10.06.81 NL 8102786**

(43) Date of publication of application:
**12.01.83 Bulletin 83/02**

(45) Publication of the grant of the patent:
**07.12.88 Bulletin 88/49**

(84) Designated Contracting States:
**AT BE DE FR GB NL**

(56) References cited:
**US-A-3 755 557**
**US-A-4 017 359**
**BIOLOGICAL ABSTRACTS, vol. 61, no. 1,
January 1976 abstract no. 2512, page 260
PHILADELPHIA (US) J.B. McFERRAN et al.:
"Studies on immunication of pigs with Bartha
strain of Aujeszky's disease virus"**
**PROC. 74th ANN. MEET. U.S. ANIMAL HEALTH
ASS., 1972; J.A. HOWARTH: "Response of
swine to virulent and modified pseudorabies
viruses", pp. 371-384**
**UNLISTED DRUGS, vol. 29, no. 10, October
1977, CATHAM, NEW JERSEY (US); page 165,
column 2, item P**

(73) Proprietor: **DUPHAR INTERNATIONAL
RESEARCH B.V**
**C.J. van Houtenlaan 36**
**NL-1381 CP Weesp (NL)**

(72) Inventor: **Zantinga, Jan Willem**
**c/o OCTROOIBUREAU ZOAN B.V., Apollolaan
151**
**NL-1077 AR Amsterdam (NL)**

(74) Representative: **Muis, Maarten et al**
**OCTROOIBUREAU ZOAN B.V. P.O. Box 140**
**NL-1380 AC Weesp (NL)**

(56) References cited:
**BIOLOGICAL ABSTRACTS, vol. 55, 15 January
1973 abstract no. 8194, page 849**
**PHILADELPHIA (US); S. NEDJALKOV et al.:
"Immunity to Aujeszky's disease in unweaned
piglets inoculated with a crystal violet
adsorbed vaccine"**

Courier Press, Leamington Spa, England.

**Description**

The invention relates to the use of live attenuated Aujeszky-virus for the manufacture of a vaccine for intranasally protecting maternally immune pigs against Aujeszky's disease.

Aujeszky's disease or pseudorabies is a generally occurring infection in many kinds of animals. In particular in pigs it is often an acute cause of death and the disease causes enormous economic losses.

In pregnant animals, Aujeszky's disease is characterized by abortion and stillbirth. In newly born pigs the disease causes a high mortality as a result of nervous affections. In breeding-pigs, later litters general suffer little from the disease because the animals are protected during the most vulernable period of their lives against the virus by maternal antibodies *via* the colostrum of latent infected sows.

On the other hand, Aujeszky's diesease in the intensive pig-fattening system where piglets originating from different places are usually put together, is an ever returning problem. Outbreaks of pseudorabies in piglets after the lactation period make this disease in the intensive pig-fattening to be one of the most important diseases of swine.

In order to protect piglets against Aujeszky's disease, a few good injection vaccines are available.

J. B. McFerran and C. Dow, for example, in Res. Vet. Sci. (1975), *19*, 17—22 describe that sero-negative piglets, that is to say piglets without maternal antibodies, can well be protected against subsequent challenge with virulent virus both by means of intramuscular injection and by intranasal administration of an Aujeszky virus (Bartha strain or K-strain) grown in Vero cells. McFerran et al, however, come to the conclusion that intranasal inoculation with the K-strain proves to be a disappointment. Since Aujeszky virus first of all multiplies in the upper part of the respiratory tract, it was expected that the local immunity after intranasal administration would be stimulated and in this manner colonisation of the upper respiratory tract by pathogenic strains would be prevented. Although a slight decrease in the degree of clinical symptoms following challenge with virulent virus could be observed after intranasal administration, these minimum advantages did not outweigh the disadvantages associated with an intranasal administration.

Good results are furthermore known, obtained by Howarth (Proc. 74th Ann. Meet, U.S. Animal Health Ass. (1972), 371—384) after intranasal administration of Aujeszky virus of the BUK-strain passaged in porcine kidney cell line. These experiments were also carried out with animals which had repeatedly been found to be sero-negative.

The disadvantage of these injection vaccines, which give good results in sero-negative animals, is that they are not active in sero-positive piglets as long as they are maternally immune. This problem particularly occurs in areas where one strives after a high titer of maternal antibodies in young animals through a good vaccination scheme of the breeding sows.

It has surprisingly been found now that piglets can be very successfully vaccinated against Aujeszky's disease already early, i.e. at an age at which they usually are still maternally immune, by vaccinating them intranasally with a living attenuated Aujeszky virus vaccine which has been passaged in mammal cells or in a continuous cell line on the basis of mammal cells.

A vaccine which is suitable for use in the method according to the invention is, for example, a vaccine which is obtained by cultivating virus of the Bartha strain (K-strain) in mammal cells or in a continuous cell line on the basis of mammal cells.

An extremely suitable vaccine according to the invention is obtained, for example, by cultivating virus of the Bartha strain in secondary porcine kidney cells or in porcine kidney continuous cell line.

It has furthermore been found that after intranasal administration of a vaccine according to the invention, a very gradual build-up of immunity takes place. In fattening pigs which are usually slaughtered at the age of approximately 26 weeks, the antibody titre goes on increasing up to the moment of slaughtering when the animals are vaccinated with a quantity of vaccine which contains $10^4$—$10^7$ $TCID_{50}$, preferably approximately $10^6$ $TCID_{50}$ of virus. In this manner the animals can be optimally protected against the disease and economic losses as a result of the disease can be minimized.

Although an early immunization and gradual build-up of immunity by means of the vaccine according to the invention is important in the first place in intensive pig-fattening systems, it was found that the new vaccine is also of great importance for the protection of breeding animals. It appeared that a very good and long standing sensibilisation of the immune system is obtained after one intranasal administration of the vaccine according to the invention. This means that the defence system in animals which have been made sensible in this way can be activated in an unprecedented degree by giving them an injection with the vaccine (booster-effect).

The invention will now be described in greater detail with reference to the ensuing experiments.

Experiment I

In this experiment, 23 piglets of 9 weeks old were used. Ten of these piglets were sero-positive (maternally immune) and the remaining 13 were sero-negative. The animals were vaccinated intranasally with $10^6$ $TCID_{50}$ of Aujeszky virus which had been cultivated in chicken embryo fibroblasts (6 sero-negative and 4 sero-positive animals), or in a continuous cell line (porcine kidney originating from the institute of Prof. Pensaert), (7 sero-negative and 6 sero-positive animals).

The results, i.e. serum neutralization titres, obtained with the two vaccines in the various groups are recorded in Tables A (sero-negative animals) and B (sero-positive animals).

Stated in Tables C and D for these groups of animals is the variation of the number of sero-negative animals at a few points of time after inoculation with the two vaccines.

From Tables A to D it appears that a vaccine produced on cell line gives a clear serologic response in sero-positive animals after one intranasal administration.

Vaccine produced in chicken embryo fibroblasts (CEF) gives a much smaller serologic response. In sero-positive animals it is only prevented that the titre drops to zero.

TABLE A
Sero-negative animals:

|  | Before vaccination | After vaccination | | | |
|---|---|---|---|---|---|
| time in weeks | 0 | 2 | 6 | 10 | 14 |
| CEF vaccines | 0 | 1,8 | 2,5 | 2,9 | 2,6 |
| Cell line vaccine | 0 | 2,2 | 4,7 | 6,8 | 5,8 |

TABLE B
Sero-positive animals:

|  | Before vaccination | After vaccination | | | |
|---|---|---|---|---|---|
| time in weeks | 0 | 2 | 6 | 10 | 14 |
| CEF vaccine | 2,0 | 3,0 | 1,3 | 1,4 | 1,5 |
| Cell line vaccine | 2,0 | 2,4 | 2,7 | 4,9 | 5,8 |

TABLE C
Variation of the number of sero-negative animals in the group which was sero-negative before vaccination:

|  | Before vaccination | After vaccination | | | |
|---|---|---|---|---|---|
| time in weeks | 0 | 2 | 6 | 10 | 14 |
| CEF vaccine | 6 | 3 | 3 | 3 | 3 |
| Cell line vaccine | 7 | 1 | 1 | 1 | 1 |

TABLE D
Variation of the number of sero-negative animals in the group which was sero-positive before vaccination:

|  | Before vaccination | After vaccination | | | |
|---|---|---|---|---|---|
| time in weeks | 0 | 2 | 6 | 10 | 14 |
| CEF vaccine | 0 | 0 | 3 | 3 | 3 |
| Cell line vaccine | 1 | 0 | 0 | 0 | 0 |

Experiment II

Ten piglets from a group of 39 ten weeks old sero-positive piglets (i.e. with maternal antibodies from the colostrum) were used as non-vaccinated control animals, 14 were vaccinated intranasally with $10^6$ $TCID_{50}$ of a vaccine (Bartha strain) cultivated in C.E.F., and the remaining 15 were vaccinated intranasally

with $10^6$ TCID$_{50}$ of a vaccine (Bartha strain) cultivated in cell line (porcine kidney). In Tables E and F are recorded the serum neutralization titres at various instants and the variation of the number of sero-negative animals in the three sub-groups, respectively.

As in Experiment I, it appears clearly that the vaccine cultivated in cell line is much more effective than the vaccine cultivated in C.E.F.

TABLE E
Sero-positive animals:

| | Before vaccination | | After vaccination | | | | |
|---|---|---|---|---|---|---|---|
| time in weeks | 3 | 0 | 2 | 4 | 8 | 12 | 15 |
| Not vaccinated | 1,6 | 1,0 | 0 | 0 | 0 | 0 | 0 |
| CEF vaccine | 1,9 | not det. | 1,2 | 1,1 | 1,3 | 1,3 | 1,5 |
| Cell line vaccine | 1,7 | not det. | 1,9 | 1,4 | 2,1 | 2,6 | 4,4 |

TABLE F
Variation of the number of sero-negative animals in the group which was sero-positive before vaccination:

| | Before vaccination | | After vaccination | | | | |
|---|---|---|---|---|---|---|---|
| time in weeks | 3 | 0 | 2 | 4 | 8 | 12 | 15 |
| Not vaccinated | 2 | 6 | 8 | 10 | 10 | 10 | 10 |
| CEF vaccine | 3 | not det. | 7 | 7 | 9 | 10 | 10 |
| Cell line vaccine | 2 | not det. | 0 | 2 | 3 | 1 | 0 |

The serum neutralization titres recorded in Tables A, B and E are stated as a serum dilution which still gives 50% neutralization of the living virus used in the experiment (100 TCID$_{50}$).

Experiment III

A group of 39 sero-positive pigments were used (12 weeks old). Nine animals served as non-vaccinated control animals. Ten animals were vaccinated intramuscularly with $10^5$ TCID$_{50}$ vaccine cultivated in C.E.F. (Bartha strain). Ten animals were vaccinated intramuscularly with $10^6$ TCID$_{50}$ vaccine cultivated in (porcine kidney) cell line (Bartha strain). Ten animals were vaccinated intranasally with $10^6$ TCID$_{50}$ vaccine cultivated in (porcine kidney) cell line (Bartha strain).

The serum neutralization titres at various instants before and after vaccination are recorded in Table G. From this it appears that after one intranasal vaccination with vaccine cultivated in cell line, the quantity of antibodies in maternal immune piglets increases considerably. This in contrast with the titres obtained after one intramuscular vaccination with a vaccine cultivated in C.E.F. and a vaccine cultivated in cell line.

TABLE G
Serum neutralization titres:

| | Before vaccination | | After vaccination | | | |
|---|---|---|---|---|---|---|
| time in weeks | 9 | 0 | 4 | 8 | 12 | 15 |
| Not vaccinated | 5,7 | 1,4 | 0 | 0 | 0 | 0 |
| 1×CEF vaccine (intramusc.) | 6,9 | 1,3 | 0,1 | 1,4 | 2,4 | 2,1 |
| 1×Cell line vacc. (intramusc.) | 4,9 | 0,1 | 0,1 | 2,2 | 2,0 | 2,2 |
| 1×Cell line vacc. (intranas.) | 5,9 | 1,3 | 1,9 | 4,8 | 6,7 | 6,7 |

Experiment IV

From a group of piglets a number were used as non-vaccinated control animals, another part was vaccinated once intramuscularly with $10^5$ $TCID_{50}$ vaccine cultivated in C.E.F., and a third part of the group was vaccinated once intranasally with $10^6$ $TCID_{50}$ vaccine cultivated in cell line in the same manner as in Experiment III.

A number of animals from each of the three sub-groups were challenged intranasally 7 weeks after the vaccination with $10^5$ $TCID_{50}$ of a virulent Aujeszky strain (NIA-3). Another part of the three sub-groups was challenged in the same manner 15 weeks after vaccination.

The number of days of growth retardation in the various groups is recorded in Table H.

Table J shows the variation of the body temperature (in °C) from 3 days before till 10 days after intranasal challenge.

TABLE H
Number of days of growth retardation after challenge

| | Challenge 7 weeks after vacc. | Challenge 15 weeks after vaccination |
|---|---|---|
| Non-vaccination control animals | 12 days | >19 days |
| 1×Intramuscular CEF vaccine | 7 days | 13,3 days |
| 1×Intranasal cell line vaccine | 0 days | 6,7 days |

5

TABLE J
Variation body temperature after challenge infection
Challenge 7 weeks after vaccination

Day

| | −3 | −2 | −1 | 0 | 1 | 2 | 3 | 4 | 5 | 6 | 7 | 8 | 9 | 10 |
|---|---|---|---|---|---|---|---|---|---|---|---|---|---|---|
| non-vaccinated control animals | 39.3 | 39.3 | 39.2 | 39.3 | 39.3 | 39.8 | *41.0* | *40.6* | *40.9* | *40.7* | *40.7* | 39.7 | 39.6 | 39.3 |
| 1×Intramuscular CEF vaccine | 39.5 | 39.5 | 39.5 | 39.6 | 39.5 | *40.0* | *40.8* | *40.6* | *40.6* | 39.5 | 39.5 | 39.5 | 39.4 | 39.3 |
| 1×Intranasal cell line vaccine | 39.4 | 39.3 | 39.4 | 39.4 | 39.4 | 39.4 | 39.8 | 39.9 | 39.6 | 39.5 | 39.3 | 39.4 | 39.4 | 39.2 |

Challenge 15 weeks after vaccination

| | −3 | −2 | −1 | 0 | 1 | 2 | 3 | 4 | 5 | 6 | 7 | 8 | 9 | 10 |
|---|---|---|---|---|---|---|---|---|---|---|---|---|---|---|
| non-vaccinated control animals | 39.5 | 39.6 | 39.6 | 39.5 | 39.7 | *40.0* | *40.8* | *41.0* | *40.7* | *40.8* | *40.3* | *40.1* | 39.8 | 39.7 |
| 1×Intramuscular CEF vaccine | 39.3 | 39.5 | 39.7 | 39.9 | 39.8 | *40.2* | *40.6* | *40.6* | *40.4* | *40.4* | *40.0* | 39.3 | 39.7 | 39.6 |
| 1×Intranasal cell line vaccine | 39.3 | 39.3 | 39.3 | 39.3 | 39.4 | 39.4 | 39.6 | *40.2* | *40.4* | 39.6 | 39.3 | 39.4 | 39.3 | 39.4 |

EP 0 069 407 B1

Experiment V

A group of 7 nine weeks old sero-positive piglets was vaccinated intranasally with a dose of $10^6$ $TCID_{50}$ of a live attenuated vaccine (Bartha strain) cultivated in porcine kidney cell line.

On the age of 34 weeks 3 of the piglets (Nos. 1, 2 and 3) were vaccinated again with $10^{5.5}$ $TCID_{50}$ of the same vaccine, however, this time by means of an intramuscular injection.

One other piglet (No. 4) was intramuscularly vaccinated with $10^{4.0}$ $TCID_{50}$ of the same vaccine.

The 3 remaining piglets (Nos. 5, 6 and 7) were injected with $10^{8.2}$ $TCID_{50}$ of inactivated vaccine.

The results, i.e., the serum neutralization titers of the seven piglets are recorded in table K. The booster-effect appears clearly from these figures.

TABLE K
Titer at the age of ... weeks

| Piglet no. | 9 | 11 | 13 | 17 | 21 | 26 | 33 |
|---|---|---|---|---|---|---|---|
| 1 | 3 | 8 | 12 | 45 | 32 | 45 | 64 |
| 2 | 4 | 22 | 89 | 64 | 64 | 45 | 128 |
| 3 | 8 | 16 | 32 | 89 | 64 | 89 | 45 |
| 4 | 6 | ⩾45 | ⩾178 | 128 | ⩾178 | 89 | 64 |
| 5 | 16 | 12 | 64 | 64 | 45 | 89 | 45 |
| 6 | 8 | 32 | 128 | 128 | 128 | 256 | 128 |
| 7 | 3 | ⩾45 | 12 | 64 | 12 | 32 | 45 |

| Piglet no. | 34 wks and 3 days | 35 | 36 | 38 | 42 | 45 | 49 |
|---|---|---|---|---|---|---|---|
| 1 | 120 | 890 | 1780 | 450 | 220 | 128 | 178 |
| 2 | 450 | 2560 | 1780 | 1280 | 640 | 178 | 256 |
| 3 | 60 | 1780 | 3550 | 1780 | 220 | 178 | 256 |
| 4 | 450 | 2560 | 7080 | 5120 | 640 | 1024 | 1412 |
| 5 | 120 | 2560 | 3550 | 2560 | 640 | 178 | 256 |
| 6 | 320 | 1780 | 2560 | 890 | 450 . | 89 | 89 |
| 7 | 220 | 640 | 890 | 320 | 160 | 128 | 178 |

**Claims**

1. Use of live attenuated Aujeszky-virus for the manufacture of a vaccine for intranasally projecting maternally immune pigs against Aujeszky's disease.

2. Use according to claim 1, characterized in that the virus of the Bartha-strain is brought into a form suitable for intranasal administration.

3. Use according to claim 2, characterized in that virus cultivated in secondary porcine kidney cells or in a porcine kidney continuous cell line is brought into a form suitable for intranasal administration.

4. Use according to claim 3, characterized in that an amount of $10^4$—$10^7$ $TCID_{50}$ of virus is brought into a form suitable for intranasal administration.

5. Use according to claim 4, characterized in that an amount of $10^6$ $TCID_{50}$ is brought into a form suitable for intranasal administration.

7

# EP 0 069 407 B1

**Patentansprüche**

1. Verwendung von lebendem attenuiertem Aujeszky-Virus zur Herstellung eines Impstoffes für intranasalen Schutz von maternal-immunen Schweinen gegen die Aujeszky-Krankheit.

2. Verwendung nach Anspruch 1, dadurch gekennzeichnet, dass Virus des Bartha-Stammes in einem für intranasale Verabreichung geeigneten Form gebracht wird.

3. Verfahren nach Anspruch 2, dadurch gekennzeichnet, dass in sekundäre Schweinniere-Zellen oder in einer kontinuierlichen Schweinniere-Zelllinie gezüchtetem Virus in einem für intranasale Verabreichung geeigneten Form gebracht wird.

4. Verwendung nach Anspruch 3, dadurch gekennzeichnet, dass eine Virusmenge von $10^4$—$10^7$ $TCID_{50}$ in einem für intranasale Verabreichung geeigneten Form gebracht wird.

5. Verwendung nach Anspruch 4, dadurch gekennzeichnet, dass eine Menge von $10^6$ $TCID_{50}$ in für intranasale Verabreidung geeigneten Form gebracht wird.

**Revendications**

1. Application de virus vivant atténué de la souche Aujeszky pour obtenir un vaccin convenant pour la protection intranasale de porcs avec immunité maternale.

2. Application tel que revendiqué dans la revendication 1, charactérisé en ce que virus de la souche Bartha est formulé convenant pour l'administration intranasale.

3. Application tel que revendiqué dans la revendication 2, charactérisé en ce que virus cultivé dans cellules secondaires de la rein de porc ou dans un ligne continue des cellules de la rein de porc est formulé convenant pour l'administration intranasale.

4. Application tel que revendiqué dans la revendication 3, characterisé en ce que une quantité de $10^4$—$10^7$ $TCID_{50}$ du virus est formulé convenant pour l'administration intranasale.

5. Application tel que revendiqué dans la revendication 4, characterisé en ce que une quantité de $10^6$ $TCID_{50}$ est formulé convenant pour l'administration intranasale.